**Europäisches Patentamt**

**European Patent Office**

**Office européen des brevets**

(19)

(11) Veröffentlichungsnummer : **0 337 210 B1**

(12)

# EUROPÄISCHE PATENTSCHRIFT

(45) Veröffentlichungstag der Patentschrift :
**10.06.92 Patentblatt 92/24**

(51) Int. Cl.$^5$ : **C07C 51/60**

(21) Anmeldenummer : **89105678.0**

(22) Anmeldetag : **31.03.89**

(54) **Verfahren zur Herstellung von Carbonsäurehalogeniden.**

(30) Priorität : **13.04.88 DE 3812175**

(43) Veröffentlichungstag der Anmeldung :
**18.10.89 Patentblatt 89/42**

(45) Bekanntmachung des Hinweises auf die
Patenterteilung :
**10.06.92 Patentblatt 92/24**

(84) Benannte Vertragsstaaten :
**BE CH DE FR GB IT LI**

(56) Entgegenhaltungen :
**DE-C- 655 683**

(56) Entgegenhaltungen :
**DE-C- 701 953**
**US-A- 1 591 364**
**SOVIET INVENTIONS ILLUSTRATED, Sektion
Ch, Woche 8441, 21. November 1984, DER-
WENT PUBLICATIONS LTD., London, E16**

(73) Patentinhaber : **BAYER AG**
**W-5090 Leverkusen 1 Bayerwerk (DE)**

(72) Erfinder : **Steffan, Guido, Dr.**
**Herzogenfeld 52**
**W-5068 Odenthal (DE)**
Erfinder : **Müller, Herbert, Dr.**
**Leopold-Gmelin-Strasse 20**
**W-5000 Köln 80 (DE)**

## Beschreibung

Die Erfindung betrifft ein Verfahren zur Herstellung von Carbonsäurehalogeniden aus den zugehörigen Carbonsäuren und einem anorganischen Säurehalogenid.

Es ist bekannt, daß organische Carbonsäuren mit Phosphorpentachlorid, Phosphoroxichlorid, Phosphortrichlorid oder Thionylchlorid unter Bildung von Carbonsäurechloriden reagieren; das Thionylchlorid ist unter den genannten Chloriden dadurch gekennzeichnet, daß es nur gasförmige Zersetzungsprodukte bildet, ist aber gleichzeitig das am wenigsten reaktionsfähige und wird daher im Überschuß eingesetzt (Organikum, 5. Auflage 1965, Seite 408, VEB Deutscher Verlag der Wissenschaften). Bei manchen Säurechloriden bereitet die spätere Abtrennung des überschüssigen Thionylchlorids jedoch Schwierigkeiten und stellt in jedem Falle einen zusätzlichen Reaktionsschritt dar, während andererseits bei Verwendung äquivalenter Mengen oder eines zu geringen Überschusses nicht umgesetzte Carbonsäure zurückbleibt und die Trennung ihrerseits erschwert. Die geringe Reaktivität ist weiterhin in vielen Fällen mit langen Reaktionszeiten und ungenügenden Ausbeuten verbunden.

Daher sind reaktionsfördernde Zusätze empfohlen worden, wie Pyridin (J. Chem. Soc. 1953, 2117) oder Carbonsäureamide wie N,N-Dimethylformamid (Helv. Chim. Acta 42 (1959), 1654). Die genannten Beschleuniger und andere haben jedoch entscheidende Nachteile, so den hohen Preis des Pyridins und seine kostspielige Wiedergewinnungsnotwendigkeit aus Umweltschutzgründen und die Cancerogenität des aus Dimethylformamid und Thionylchlorid entstehenden N,N-Dimethylcarbamoylchlorids (C.A. 75, 48.340m; C.A. 77, 97.540b). Es ist weiterhin schwierig, geringste Reste dieser Beschleuniger aus dem Reaktionsprodukt zu entfernen, wie dies bereits für überschüssiges Thionylchlorid geschildert wurde. Für Phosgen, das ohne einen Beschleuniger im allgemeinen nur mangelhaft oder überhaupt nicht reagiert, gilt Entsprechendes. Reste solcher organischer Beschleuniger können die (katalytische) Weiterverwendung der Carbonsäurechloride stark beeinträchtigen.

Es wurde nun ein Verfahren zur Herstellung von Carbonsäurehalogeniden aus Carbonsäuren der Formel

$$R\text{-COOH},$$

worin

R $C_7$-$C_{13}$-Aralkyl, $C_6$-$C_{12}$-Aryl oder $R^1$-$(CH_2)n$- bedeutet, wobei $R^1$ für einen heterocyclischen Rest steht und n den Wert 0 - 4 annimmt,

und einem anorganischen Säurehalogenid der Formel

$$AOX_2 \quad (I),$$

in der

A Kohlenstoff oder Schwefel und

X Chlor oder Brom bedeuten,

in einer Menge von 0,9 bis 1,5 Mol pro Äquivalent zu bildender Carbonsäurehalogenidgruppe bei 40 bis 180°C gefunden, das dadurch gekennzeichnet ist, daß die Umsetzung in flüssiger Phase und in Gegenwart eines Magnesium-ähnlichen Metalls aus der Gruppe von Magnesium, Scandium und Zirkonium, das sich in der halogenierten Form befindet oder sich während der Reaktion in die halogenierte Form überführen läßt und in seiner Einsatzform in einer Menge von 0,1 bis 30 g pro Mol der Carbonsäure eingesetzt wird, durchgeführt wird.

Im erfindungsgemäßen Verfahren können beispielsweise Carbonsäuren der allgemeinen Formel

$$R\text{-COOH} \quad (II)$$

umgesetzt werden, in der

R $C_{13}$-Aralkyl, $C_6$-$C_{12}$-Aryl oder $R^1$-$(CH_2)_n$- bedeutet,

worin $R^1$ für einen heterocyclischen Rest steht und n den Wert 0-4 annimmt.

Aralkyl bedeutet beispielsweise Benzyl, Phenyl-ethyl, Naphthyl-methyl oder Biphenylmethyl; bevorzugt Benzyl; Aryl bedeutet Phenyl, Naphthyl oder Biphenyl, bevorzugt Phenyl. Der heterocyclische Rest bedeutet einen 5- oder 6-Ring mit einem oder zwei O-, N- und/oder S-Atomen im Ring, der aromatisch oder nicht aromatisch sein kann, wie 2- oder 3-Furyl, 2- oder 3-Thienyl, 2- oder 3-Pyrrolyl, 2- oder 4-Imidazolyl, 2- oder 3-Oxazolyl, 2- oder 3-Thiazolyl, Pyridinyl, Morpholyl, Thiomorpholyl, Pyrazolyl oder andere.

Die genannten Reste können weiterhin ein- oder zweifach substituiert sein. Als Substituenten seien genannt: $C_1$-$C_4$-Alkylsubstituenten, wie Methyl, Ethyl, Propyl, Isopropyl, Butyl, Isobutyl; $C_1$-$C_4$-Alkoxy, wie Methoxy, Ethoxy, Propoxy, Isopropoxy, Butoxy, Isobutoxy; Halogen, wie Fluor, Chlor oder Brom; Nitro, Cyano, Dialkylamino mit $C_1$-$C_4$-Alkylgruppen; $C_2$-$C_4$-Acyl, wie Acetyl, Propionyl, Butyryl; $C_2$-$C_4$-Acyloxy, wie Acetoxy, Propionyloxy, Butyryloxy; Carboxyl. Damit gelangt man unter anderem zu verzweigten Carbonsäuren bzw. zu Di- und Tricarbonsäuren.

Als anorganische Säurehalogenide kommen in Frage: Phosgen, Carbonyldibromid, Thionylchlorid, Thionylbromid. In bevorzugter Weise seien Phosgen und Thionylchlorid genannt.

Das anorganische Säurehalogenid wird in einer Menge von 0,9-1,5 Mol, bevorzugt 1,1-1,2 Mol, pro Mol Carbonsäure eingesetzt. Für den Fall, daß die umzusetzende Carbonsäure mehr als eine Carboxylgruppe enthält, werden die genannten Zahlen mit der Zahl der Carboxylgruppen multipliziert.

Das erfindungsgemäße Verfahren wird bei einer Temperatur von 40-180°C, bevorzugt 70-180°C, besonders bevorzugt 80-150°C, ganz besonders bevorzugt 90-130°C durchgeführt; insbesondere im tieferen Temperaturbereich ergeben sich für empfindliche und niedrig siedende Substrate Vorteile in Bezug auf Reinheit und höhere Ausbeute.

Das erfindungsgemäße Verfahren kann in Gegenwart von inerten Lösungsmitteln, beispielsweise in einer Menge von 100 bis 1000 Gew.-% eines solchen inerten Lösungsmittels, bezogen auf die Menge Carbonsäure, durchgeführt werden. Als inerte Lösungsmittel kommen beispielsweise in Frage: aliphatische und aromatische Kohlenwasserstoffe geeigneter Siedepunktslage, um bevorzugt druckfrei arbeiten zu können, wie Cyclohexan, Isododecan, Petrolether, Kerosin, Benzol, Toluol, Xylol; aliphatische und aromatische Halogenkohlenwasserstoffe, wie Chloroform, Ethylidendichlorid, Ethantetrachlorid, Chlorbenzol, Dichlorbenzol.

Sofern die umzusetzende Carbonsäure bzw. das Säurehalogenid ein flüssiges Reaktionsmedium gewährleisten, kann auf ein Lösungsmittel verzichtet werden. Wenn es sich in diesem Falle um hochschmelzende Substrate handelt, wird im oberen Bereich der angegebenen Temperaturen gearbeitet. Für den Fall, daß das herzustellende Säurehalogenid unter den Reaktionsbedingungen flüssig ist, kann solches Säurehalogenid, etwa aus einer früheren Charge, vorgelegt werden und als Ersatz für ein sonstiges Lösungsmittel als Reaktionsmedium dienen.

Erfindungsgemäß wird die Herstellung der Carbonsäurehalogenide in Gegenwart eines Magnesium-ähnlichen Metalls in halogenierter Form durchgeführt. Solche Metalle sind neben Magnesium weiterhin Scandium und Zirkonium. Alle diese Metalle sind durch die dem Fachmann bekannte "Schrägbeziehung" im Periodensystem der Elemente miteinander verwandt. Bevorzugt wird Magnesium eingesetzt.

Die genannten Metalle wirken in ihrer halogenierten Form, beispielsweise als Fluoride, Chloride, Bromide oder Jodide, bevorzugt als Chloride. Es wirken jedoch auch Formen, die sich während der erfindungsgemäßen Durchführung in die halogenierte Form überführen lassen. Hierzu seien genannt: Die Oxide, Hydroxide, Sulfate, Nitrate, Carbonate, Hydrogencarbonate, Acetate und andere anorganische und organische Salze der genannten Metalle, die sich unter den erfindungsgemäßen Bedingungen in die halogenierte Form überführen lassen. Grundsätzlich läßt sich sogar die metallische Form in die halogenierte Form überführen.

Die bereits in der halogenierten Form vorliegenden Metalle verkürzen vielfach die Reaktionszeit und sind daher bevorzugt. Sie können in wasserhaltiger oder teilweise oder ganz entwässerter Form vorliegen. Besonders bevorzugt ist der Einsatz von Magnesium chlorid beispielsweise als $MgCl_2 \cdot 6H_2O$.

Erfindungsgemäß wird das Magnesium-ähnliche Metall, das sich in der halogenierten Form befindet oder sich während der Reaktion in die halogenierte Form überführen läßt, in einer Menge von 0,1-30 g, bevorzugt 0,2-20 g, besonders bevorzugt 0,3-10 g, pro Mol der Carbonsäure eingesetzt. Das Magnesium-ähnliche Metall kann auch auf einen Träger, wie Aktivkohle oder Kieselsäure, aufgebracht oder mit diesem zusammen eingesetzt werden.

Die im erfindungsgemäßen Verfahren herstellbaren Carbonsäurehalogenide können vom eingesetzten Magnesium-ähnlichen Metall in halogenierter Form abdestilliert oder abfiltriert oder im einfachsten Fall sogar dekantiert werden, ohne daß Verunreinigungen in das Carbonsäurehalogenid geraten. Die Carbonsäurehalogenide sind daher besonders rein und farblos und für eine weitere Verarbeitung besonders gut geeignet; sie sind weiterhin frei von gesundheitsschädlichen Nebenprodukten und Beschleunigern, etwa frei von denen des Standes der Technik.

Außerdem lassen sich dadurch die Katalysatoren in vielen Fällen vielfach wiederverwenden, so daß der Verbrauch extrem gering wird. Sie sind hier also als echte Katalysatoren zu verstehen.

Carbonsäurehalogenide sind dem Fachmann bekannte Substanzen, die beispielsweise für Acylierungen der verschiedensten Art eingesetzt werden können.

Beispiel 1

180 g (1 Mol) p-Acetoxy-benzoesäure, 400 g Xylol und 3 g $MgCl_2 \cdot 6H_2O$ wurden vorgelegt. Bei einer Temperatur von 100°C wurden sodann innerhalb von etwa 4 Stunden etwa 160 g (etwa 1,6 Mol) Phosgen eingeleitet. Nach gaschromatographischer Untersuchung war danach der Umsatz der vorgelegten Säure vollständig. Das $MgCl_2 \cdot 6H_2O$ wurde abfiltriert; es konnte für einen weiteren Ansatz wieder verwendet werden. Das Filtrat wurde destillativ aufgearbeitet und ergab das p-Acetoxy-benzoylchlorid in 98 % der theoretischen Ausbeute.

Beispiel 2

120 g (1 Mol) Benzoesäure, 400 g Xylol und 3 g $MgCl_2 \cdot 6H_2O$ wurden vorgelegt. Bei einer Temperatur von

3

100°C wurden innerhalb von etwa 4 Stunden etwa 165 g (1,7 Mol) Phosgen eingeleitet. Nach gaschromatographischer Analyse war der Umsatz danach vollständig. Das Reaktionsgemisch wurde wie in Beispiel 1 aufgearbeitet und ergab Benzoylchlorid in 98 % der theoretischen Ausbeute.

Beispiel 2a (zum Vergleich)

Zum Vergleich wurde der Versuch nach Beispiel 2 ohne Zusatz von Magnesiumchlorid wiederholt. Nach Einleiten von Phosgen setzte die Abgasentwicklung nur zögernd ein. Phosgen sammelte sich am Rückflußkondensator. Nach Einleiten von ca. 100 g (ca. 1 Mol) Phosgen sank die Reaktionstemperatur stark ab infolge der Anreicherung von Phosgen. Auch nach Erhöhen der Heizbadtemperatur auf 140°C erfolgte kein weiterer Umsatz. Nach gaschromatografischer Analyse des Gemisches hatte sich etwa 1/3 Benzoylchlorid neben etwa gleichen Mengen Benzoesäure und Benzoesäureanhydrid gebildet.

Beispiel 3

1000 g (etwa 8,4 Mol) Thionylchlorid, 540 g (3 Mol) p-Acetoxy-benzoesäure und 5 g $MgCl_2 \cdot 6H_2O$ wurden vorgelegt. Anschließend wurde die Temperatur vorsichtig von 30°C bis 95°C so erhöht, daß eine gleichmäßige, gut zu überwachende Gasentwicklung eingehalten wurde; bis zum Ende der Gasentwicklung benötigte man hierzu 4 Stunden. Anschließend wurde der Überschuß an Thionylchlorid abdestilliert, vom $MgCl_2$ dekantiert und destilliert. Es wurden 98 % der theoretischen Ausbeute an p-Acetoxy-benzoylchlorid erhalten.

Beispiel 4 (zum Vergleich)

Beispiel 3 wurde wiederholt, wobei jedoch ohne Zusatz von $MgCl_2 \cdot 6H_2O$ gearbeitet wurde. Bis zum Ende der Gasentwicklung benötigte man ca. 12 Stunden; die Ausbeute betrug lediglich 90 % der theoretischen Ausbeute.

Beispiele 5-20 (Beispiele 19-22 zum Vergleich)

Diese Beispiele wurden nach folgender allgemeiner Vorschrift durchgeführt:
Einsatzmengen :
    1 Mol R-COOH
    400 g Lösungsmittel
    3 g Mg-Verbindung
    Phosgen bis zur Sättigung
Durchführung: Die Carbonsäure, das Lösungsmittel und die Mg-Verbindung wurden vorgelegt und auf die Reaktionstemperatur erwärmt. Dann wurde Phosgen eingeleitet; das Abgas wurde durch einen mit Trockeneis beschickten Rückflußkühler geleitet, so daß nicht umgesetztes Phosgen in das Reaktionsgemisch zurücklief. Das Ende der Reaktion war durch verstärkte Phosgenkondensation und nachlassende Abgasentwicklung erkennbar. Die folgende Tabelle faßt alle Daten zusammen.

Tabelle: Phosgenierung von Carbonsäuren R-COOH in Gegenwart von Mg-Verbindungen

| Nr. | R | Lösungsmittel | Mg-Verb. | Temp. ($^0$C) | Zeit (h) | Ausbeute (%) |
|---|---|---|---|---|---|---|
| 5 | $3-NO_2-C_6H_4-$ | Xylol | " | 100 | 1 | 91 |
| 6 | $3-CH_3-C_6H_4-$ | " | " | 100 | 3 | 96 |
| 7 | $4-CH_3COO-C_6H_4-$ | " | $Mg(OH)_2$ | 100 | 5 | 98 |
| 8 | " | " | $MgSO_4 \cdot 7H_2O$ | 100 | 6 | 91 |
| 9 | " | " | $MgCl_2 \cdot 6H_2O$ | 100 | 1 | 98 |
| 10 | " | " | $Mg(CH_3COO)_2 \cdot 4H_2O$ | 100 | 7 | 98 |
| 11 | " | " | $Mg(NO_3)_2 \cdot 6H_2O$ | 100 | 7 | 90 |
| 12 | " | " | $MgO$ | 100 | 5 | 98 |
| 13 | " | " | $SnCl_2$ | 100 | keine Reaktion | |
| 14 | " | " | $AlCl_3$ | 100 | keine Reaktion | |

EP 0 337 210 B1

Tabelle: Phosgenierung von Carbonsäuren R-COOH in Gegenwart von Mg-Verbindungen

| Nr. | R | Lösungsmittel | Mg-Verb. | Temp. (°C) | Zeit (h) | Ausbeute (%) |
|---|---|---|---|---|---|---|
| 15 | $4-CH_3COO-C_6H_4-$ | Xylol | $FeCl_3$ | 100 | keine Reaktion | |
| 16 | " | " | $CaCl_2$ | 100 | keine Reaktion | |
| 17 | " | Cl-benzol | $MgCl_2 \cdot 6H_2O$ | 100 | 1,5 | 93 |
| 18 | 2-Furyl | " | $MgCl_2 \cdot 6H_2O$ | 100 | 4 | 93 |
| 19 | 2-Thienyl | " | $MgCl_2 \cdot 6H_2O$ | 100 | 3 | 91 |
| 20 | 4-Chlor-1H-pyrazolyl | " | $MgCl_2 \cdot 6H_2O$ | 100 | 1,5 | 96 |

EP 0 337 210 B1

### Beispiel 21

Nach der Vorschrift der Beispiele 5-20 wurde Benzoesäure in Xylol in Gegenwart von $MgCl_2 \cdot 6H_2O$ bei 100°C umgesetzt. In 4 Stunden erfolgte quantitative Umsetzung.

### Beispiel 22 (zum Vergleich)

Beispiel 21 wurde ohne $MgCl_2 \cdot 6H_2O$ wiederholt. Auch bei Temperaturen bis zu 140°C blieb die Reaktion bei kleinen Umsätzen stehen.

### Beispiel 23

Die Apparatur bestand aus einem 0,5 1-Vierhalskolben mit Trockeneis-Tiefkühler, Rührer, Heizbad, $COCl_2$-Stahlflasche auf einer Waage, verbunden mit einer getauchten Einleitung, Abgasableitung auf einen A-Kohle-Wäscher zur $COCl_2$-Vernichtung sowie Abgaskontrollen.

Vorgelegt wurden 102 g (0,5 Mol) Isophthalsäuredichlorid und 102 g (0,5 Mol) Terephthalsäuredichlorid als Reaktionsmedium, ferner 83 g (0,5 Mol) Isophthalsäure, 83 g (0,5 Mol) Terephthalsäure und 3 g (0,015 Mol) $MgCl_2 \cdot 6H_2O$. Dazu gab man bei 160°C innerhalb von 7 Stunden 250 g (2,5 Mol) Phosgen.

Die anfangs gut rührbare Suspension wurde nach Zugabe von ca. 100 g $COCl_2$ dickflüssiger; nach Zugabe von ca. 210 g $COCl_2$ erhielt man eine klare Lösung. Am Ende der $COCl_2$-Einleitung kommt überschüssiges $COCl_2$ zum Rückfluß; die Sumpftemperatur fiel dabei auf 150°C oder etwas darunter. Man ließ 30 Min. nachrühren und blies das überschüssige $COCl_2$ mit $N_2$ aus.

Man erhielt 389 g einer Schmelze mit einem Erstarrungspunkt von ca. 56°C. Die gaschromatographische Untersuchung ergab 48,4 % Terephthalsäuredichlorid und 51,6 % Isophthalsäuredichlorid an Säurechloriden bei einer Gesamtausbeute von 95,8 % der theoretischen Ausbeute.

### Beispiel 24

In der Apparatur von Beispiel 23 wurden 203 g (1 Mol) Isophthalsäuredichlorid, 166 g (1 Mol) Isophthalsäure und 3 g (0,015 Mol) $MgCl_2 \cdot 6H_2O$ vorgelegt und bei 160°C innerhalb von 7 Stunden ca. 250 g (2,5 Mol) $COCl_2$ eingeleitet. Die zunächst gut rührbare Suspension wurde nach Einleiten von ca. 120 g $COCl_2$ eine klare Lösung, verdickte sich nach Einleiten von ca. 170 g $COCl_2$ und wurde nach Einleiten von ca. 200 g $COCl_2$ wieder dünn-flüssig. Am Ende der $COCl_2$-Einleitung trat $COCl_2$-Rückfluß auf, woraufhin die Sumpftemperatur auf ca. 150°C oder tiefer abfiel. Nach 30 Min. Nachrührzeit wurde überschüssiges $COCl_2$ mit $N_2$ ausgeblasen. Man erhielt 388 g Schmelze mit einem Erstarrungspunkt von 44-45°C. Die Ausbeute betrug 95,5 % der theoretischen Ausbeute.

## Patentansprüche

1. Verfahren zur Herstellung von Carbonsäurehalogeniden aus Carbonsäuren der Formel

$$R\text{-}COOH,$$

worin

R $C_7$-$C_{13}$-Aralkyl, $C_6$-$C_{12}$-Aryl oder $R^1$-$(CH_2)_n$ bedeutet,

wobei $R^1$ für einen heterocyclischen Rest steht und n den Wert 0 bis 4 annimmt,

und einem anorganischen Säurehalogenid der Formel

$$AOX_2,$$

in der

A Kohlenstoff oder Schwefel und

X Chlor oder Brom bedeuten,

in einer Menge von 0,9 - 1,5 Mol pro Äquivalent zu bildender Carbonsäurehalogenidgruppe bei 40 bis 180°C , dadurch gekennzeichnet, daß die Umsetzung in flüssiger Phase und in Gegenwart eines Magnesium-ähnlichen Metalles aus der Gruppe von Magnesium, Scandium und Zirkonium, das sich in der halogenierten Form befindet oder sich während der Reaktion in die halogenierte Form überführen läßt und in seiner Einsatzform in einer Menge von 0,1 - 30 g pro Mol der Carbonsäure eingesetzt wird, durchgeführt wird.

2. Verfahren nach Anspruch 1, dadurch gekennzeichnet, daß als Magnesium-ähnliches Metall Magnesium eingesetzt wird.

3. Verfahren nach Anspruch 1, dadurch gekennzeichnet, daß die Magnesium-ähnlichen Metalle als Halogenide eingesetzt werden.

4. Verfahren nach Anspruch 3, dadurch gekennzeichnet, daß MgCl$_2$·6H$_2$O eingesetzt wird.

5. Verfahren nach Anspruch 1, dadurch gekennzeichnet, daß das Magnesium-ähnliche Metall in seiner Einsatzform in einer Menge von 0,2-20 g, bevorzugt 0,3-10 g pro Mol der Carbonsäure eingesetzt wird.

6. Verfahren nach Anspruch 1, dadurch gekennzeichnet, daß bei einer Temperatur von 70-180°C, bevorzugt 80-150°C, besonders bevorzugt 90-130°C, gearbeitet wird.

7. Verfahren nach Anspruch 1, dadurch gekennzeichnet, daß in Gegenwart von 100-1000 Gew.-% eines inerten Lösungsmittels, bezogen auf die Menge Carbonsäure, gearbeitet wird.

8. Verfahren nach Anspruch 1, dadurch gekennzeichnet, daß 1,1-1,2 Mol anorganisches Säurechlorid, pro Äquivalent zu bildender Carbonsäurehalogenidgruppe eingesetzt werden.

9. Verfahren nach Anspruch 1, dadurch gekennzeichnet, daß als anorganisches Säurehalogenid Phosgen oder Thionylchlorid eingesetzt wird.

## Claims

1. Process for the preparation of carboxylic acid halides from carboxylic acids of the formula

$$R\text{-COOH,}$$

in which

R denotes C$_7$-C$_{13}$-aralkyl, C$_6$-C$_{12}$-aryl or R$^1$-(CH$_2$)$_n$,

where R$^1$ represents a heterocylic radical and n adopts the value 0 to 4,

and an inorganic acid halide of the formula

$$AOX_2,$$

in which

A denotes carbon or sulphur and

X denotes chlorine or bromine,

in an amount of 0.9 - 1.5 mol per equivalent of carboxylic acid halide group to be formed, at 40 to 180°C, characterised in that the reaction is carried out in the liquid phase and in the presence of a magnesium-like metal from the group consisting of magnesium, scandium and zirconium, which metal is in the halogenated form or can be converted into the halogenated form during the reaction and in the form in which it is employed is used in an amount of 0.1 - 30 g per mole of the carboxylic acid.

2. Process according to Claim 1, characterised in that the magnesium-like metal used is magnesium.

3. Process according to Claim 1, characterised in that the magnesium-like metals are used as halides.

4. Process according to Claim 3, characterised in that MgCl$_2$·6H$_2$O is used.

5. Process according to Claim 1, characterised in that the magnesium-like metal in the form in which it is employed is used in an amount of 0.2 - 20 g, preferably 0.3 - 10 g, per mole of the carboxylic acid.

6. Process according to Claim 1, characterised in that the reaction is carried out at a temperature of 70 -180°C, preferably 80 - 150°C, particularly preferably 90 - 130°C.

7. Process according to Claim 1, characterised in that the reaction is carried out in the presence of 100 -1000 % by weight of an inert solvent, relative to the amount of carboxylic acid.

8. Process according to Claim 1, characterised in that 1.1 - 1.2 mol of inorganic acid chloride are used per equivalent of carboxylic acid halide group to be formed.

9. Process according to Claim 1, characterised in that the inorganic acid halide used is phosgene or thionyl chloride.

## Revendications

1. Procédé de production d'halogénures d'acides carboxyliques à partir d'acides carboxyliques de formule

$$R\text{-COOH,}$$

dans laquelle

R est un groupe par alkyle en C$_7$ à C$_{13}$, aryle en C$_6$ à C$_{12}$ ou R$^1$-(CH$_2$)$_n$, où R$^1$ est un reste hétérocyclique et n a une valeur de 0 à 4,

et d'un halogénure d'acide inorganique de formule

$$AOX_2,$$

dans laquelle

A représente le carbone ou le soufre et

X représente le chlore ou le brome,

en une quantité de 0,9 à 1,5 mole par équivalent de groupe halogénure d'acide carboxylique à former, à une température de 40 à 180°C, caractérisé en ce qu'on conduit la réaction en phase liquide et en présence d'un métal analogue au magnésium choisi dans le groupe comprenant le magnésium, le scandium et le zirconium, qui se trouve sous la forme halogénée ou qui peut être converti en la forme halogénée pendant la réaction et qui est utilisé sous sa forme introduite en une quantité de 0,1 à 30 g par mole d'acide carboxylique.

2. Procédé suivant la revendication 1, caractérisé en ce qu'on utilise le magnésium comme métal analogue au magnésium.

3. Procédé suivant la revendication 1, caractérisé en ce qu'on utilise les métaux analogues au magnésium sous forme d'halogénures.

4. Procédé suivant la revendication 3, caractérisé en ce qu'on utilise $MgCl_2 \cdot 6H_2O$.

5. Procédé suivant la revendication 1, caractérisé en ce qu'on utilise le métal analogue au magnésium sous sa forme introduite en une quantité de 0,2 à 20 g, de préférence de 0,3 à 10 g par mole d'acide carboxylique.

6. Procédé suivant la revendication 1, caractérisé en ce qu'on opère à une température de 70 à 180°C, de préférence de 80 à 150°C, notamment de 90 à 130°C.

7. Procédé suivant la revendication 1, caractérisé en ce qu'on opère en présence de 100 à 1000% en poids d'un solvant inerte, par rapport à la quantité d'acide carboxylique.

8. Procédé suivant la revendication 1, caractérisé en ce qu'on utilise 1,1 à 1,2 mole de chlorure d'acide inorganique par équivalent de groupe halogénure d'acide carboxylique à former.

9. Procédé suivant la revendication 1, caractérisé en ce qu'on utilise comme halogénure d'acide inorganique le phosgène ou le chlorure de thionyle.